# EUROPÄISCHE PATENTSCHRIFT

(11) **EP 1 791 596 B1**
(45) Veröffentlichungstag und Bekanntmachung des Hinweises auf die Patenterteilung: **02.01.2008**
(21) Anmeldenummer: 05808254.6
(22) Anmeldetag: 09.09.2005
(51) Int. Cl.: A61N 5/10

(54) **NACHLADEVORRICHTUNG ZUR BRACHYTHERAPIE VON TUMOREN**
AFTERLOADING DEVICE FOR BRACHYTHERAPY OF TUMOURS
APPAREIL PROJECTEUR DE SOURCES POUR LA BRACHYTHERAPIE DE TUMEURS

(30) Priorität: 10.09.2004 DE 102004043880
(43) Veröffentlichungstag der Anmeldung: 06.06.2007
(73) Patentinhaber: Puthawala, Anwer, 91054 Buckenhof (DE)
(72) Erfinder: Puthawala, Anwer, 91054 Buckenhof (DE)
(74) Vertreter: Blaumeier, Jörg
(86) Internationale Anmeldenummer: PCT/EP2005/009691
(87) Internationale Veröffentlichungsnummer: WO 2006/027254

(56) Entgegenhaltungen:
- EP-A- 0 128 300
- EP-A- 0 340 881
- DE-A1- 3 442 762
- DE-A1- 3 640 790
- DE-A1- 4 123 501
- US-A- 5 139 473

## Beschreibung

Durch DE 34 42 762 C2 ist eine Nachladevorrichtung zur Brachytherapie von Tumoren bekannt, bei der mehrere Rohre vorgesehen sind, in denen radioaktive Strahlenquellen an Seilen oder Stangen magnetisch längsverschiebbar sind. Diese Strahlenquellen können über einen Kopplungsschieber in Schläuche eingeführt werden, die zum Tumor führen.

Für jede Bestrahlung müssen ganz bestimmte Strahlenquellen angewendet werden. In der Vorrichtung ist eine Vielzahl von Strahlenquellen auf jeweils einem Seil vorgesehen und die jeweils erforderlichen Strahlenquellen werden durch die Schläuche in den Patienten gebracht. Soll z. B. eine Bestrahlung mit hoher oder mit niedriger Dosis durchgeführt werden, so ist dies mit der oben genannten Vorrichtung schwer realisierbar.

Der Erfindung liegt die Aufgabe zu Grunde, eine Vorrichtung der eingangs genannten Art gegenüber dem Stand der Technik so zu vereinfachen, dass ein Wechsel und auch eine schrittweise Verschiebung der Strahlenquelle am Behandlungsort einfach und schnell möglich ist.

Diese Aufgabe ist erfindungsgemäß gelöst durch die Merkmale des Patentanspruchs 1.

Bei der Erfindung können in mehreren Rohren unterschiedliche Strahlenquellen mit unterschiedlichen Aktivitäten untergebracht werden. Durch Verstellen des Verteilers kann die jeweils freigegebene Strahlenquelle in den gewünschten Schlauch, der zum Patienten führt, eingeführt werden, ohne dass aufwendige Wechsel- und Austauschvorgänge erforderlich sind.

Verstellbare Verteiler sind auch bekannt aus US 5,139,437, DE 4 123 501 A1, DE 36 40 790. Diese Verteiler sind jedoch nicht abnehmbar.

Vorteilhafte Weiterbildungen der Erfindung ergeben sich aus den Unteransprüchen.

Eine besonders zweckmäßige Weiterbildung ergibt sich aus dem Patentanspruch 7. Durch die Krümmung der Rohre ergibt sich gegenüber dem Stand der Technik, bei dem die Rohre langgestreckt sind, ein besonders kompakter und platzsparender Aufbau.

Die Erfindung ist nachfolgend anhand der Zeichnung näher erläutert. Es zeigen:
- Fig. 1 und 2: eine Seitenansicht und Frontansicht einer Vorrichtung nach der Erfindung,
- Fig. 3 und 4: Details aus der Fig. 1 an den Stellen I und II,
- Fig. 5: eine Variante der Vorrichtung gemäß den Fig. 1 bis 3 in Seitenansicht und
- Fig. 6: eine besonders platzsparende Ausführung einer Vorrichtung nach der Erfindung.

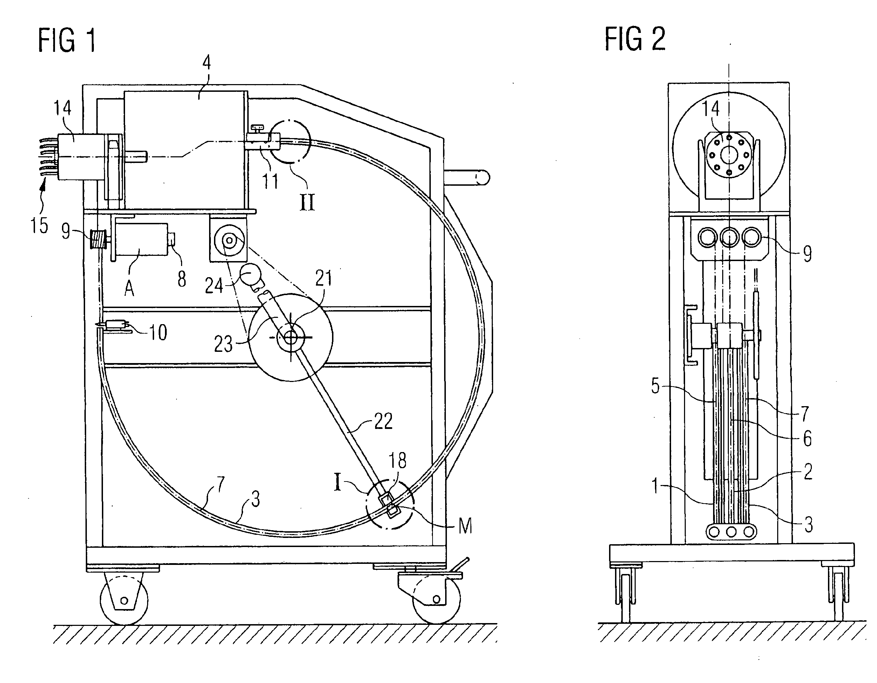

In den Fig. 1 und 2 sind mit 1, 2, 3 drei Rohre aus nicht magnetisierbarem Material, z.B. rostfreiem Stahl, bezeichnet, die teilkreisförmig gekrümmt sind, wobei zwischen den beiden Enden jedes Rohres jeweils etwa ein Viertelkreis liegt. Am oberen Ende der Nachladevorrichtung liegt eine Abschirmung 4, zu der die oberen Enden der Rohre 1, 2, 3 geführt sind. Im Innern der Rohre 1, 2, 3 ist jeweils ein flexibles Seil 5, 6, 7 längsverschiebbar geführt.

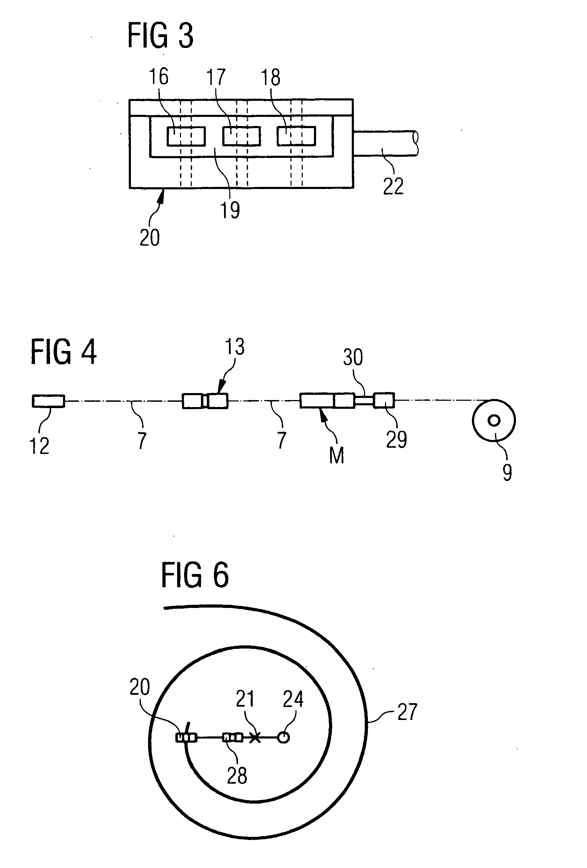

Die Seile 5, 6, 7 sind durch strichpunktierte Linien dargestellt. Zur Messung des von den Seilen 5, 6, 7 und damit von den Strahlenquellen jeweils zurückgelegten Weges dienen Impulsgeber, von denen einer in Fig. 1 dargestellt und mit 8 bezeichnet ist. Die Impulsgeber sind mit Antriebsmotoren verbunden, von denen der Antriebsmotor A in Fig.1 sichtbar ist. Die Seile 5, 6, 7 sind auf Spulenkörpern wendelförmig aufgewickelt. Der Spulenkörper des Impulsgebers 8 ist in Fig. 1 mit 9 bezeichnet. Für jedes Seil 5, 6, 7 ist eine elektrische Klemmvorrichtung vorgesehen, die das jeweils nicht zu verstellende Seil festhält und von denen in Fig. 1 die Klemmvorrichtung 10 für das Seil 7 gezeigt ist. Zwischen der Abschirmvorrichtung 4 und den oberen Enden der Rohre 1, 2, 3 ist eine Wechselvorrichtung 11 für die Strahlenquellen vorgesehen. Die Strahlenquellen, von denen in Fig. 4 eine gezeigt und mit 12 bezeichnet ist, werden an den oberen Enden der Seile 5, 6, 7, z.B. bei zwei Strahlenquellen 12 der Seiten 5 und 7, angebracht. Die Fig. 4 zeigt, dass die jeweilige Strahlenquelle, z.B. 12, mit dem jeweiligen Seil, z.B. 7, über eine Schraubverbindung 13 verbunden ist, deren einer Teil drehbar am Ende des Seiles 7 und deren anderer Teil an einem Seilstück, das die Strahlenquelle 12 trägt, angebracht ist. An Stelle der Schraubverbindung kann auch eine Steckverbindung vorgesehen werden.

Ein Verteiler 14 weist Schläuche 15 auf, die in den zu behandelnden Tumor über Kopplungsstücke einführbar sind. Er ist abnehmbar an der Abschirmung 4 gelagert, so dass er in Bestrahlungspausen vom Patienten getragen werden kann. Der Verteiler 14 ist verstellbar (drehbar) und der Eingang des jeweils gewünschten Schlauches 15 kann auf den Ausgang eines der Rohre 1, 2, 3 ausgerichtet werden. Die jeweils gewünschte Strahlenquelle 12 oder ein Dummy können so in den Verteiler 14 eingeführt werden. Die Abschirmung 4 und die Rohre 1, 2, 3 sind so ausgerichtet, dass die Seile 5, 6, 7 etwa horizontal zum Verteiler 14 geführt werden.

Zur Längsverschiebung der Seile 5, 6, 7 sind die Rohre 1, 2, 3 von Dauermagneten 16, 17, 18 (Fig. 3) mit zentrischen Löchern umschlossen, die magnetisch mit Magnetelementen aus magnetisierbarem Material auf den Seilen 5, 6, 7 gekoppelt und auf den Rohren 1, 2, 3 verschiebbar sind. In Fig. 1 ist das Magnetelement des Seiles 7 mit M bezeichnet. Auch eine andere Magnetkonfiguration, z.B. zwei Flachmagnete, erfüllen die gewünschte Funktion.

Die Dauermagnete 16, 17, 18 (Fig. 3) sind im Hohlraum 19 eines Halters 20 gelagert, der am Ende eines um den Kreismittelpunkt 21 der Rohre 1, 2, 3 drehbaren Armes 22 befestigt ist. Die Dauermagnete 16, 17, 18 sind zum Ausgleich von Krümmungstoleranzen der Rohre 1, 2, 3 im Halter 20 quer zur Rohr-Längsrichtung zweidimensional verschiebbar gelagert. An einer über den Drehpunkt des Armes 22 hinausragenden Verlängerung 23 des Armes 22 ist ein Ausgleichsgewicht 24 angebracht, damit eine exakte schrittweise Verstellung der jeweiligen Strahlenquelle möglich ist.

In den Rohren 1, 2, 3 werden unterschiedliche Strahlenquellen gelagert. Die Tumorbehandlung geschieht in der Weise, dass einer der Schläuche 15 über ein Kupplungsstück in den Tumor eingeführt und der Verteiler 14 so eingestellt wird, dass die gewünschte Strahlenquelle in den durch Einstellung des Verteilers 14 gewählten Eingang für diesen Schlauch eingeführt werden kann. Dann wird der Arm 22 motorisch entgegen dem Uhrzeigersinn gedreht, wobei nur das Seil mit der richtigen Strahlenquelle mitgenommen wird, weil die anderen Seile durch die jeweiligen Klemmvorrichtungen 10 (in Fig. 2 nicht gezeigt) arretiert werden. Es können z.B. in den Rohren 1, 2, 3 eine Strahlenquelle für hohe Dosis (high dose), eine für niedrige Dosis (low dose) und ein Dummy (nicht strahlender Stift) für Testzwecke untergebracht werden. In dem Beispiel kann der Dummy mit dem Ende des Seiles 6 verbunden sein. Für den Dummy ist ein Impulsgeber 8 und ein Antriebsmotor nicht unbedingt erforderlich. Auch eine Strahlenquelle für Pulsdosis (pulse dose), kann wahlweise vorgesehen werden, die eine mehrmalige Bestrahlung mit bestrahlungsfreien Intervallen erlaubt. Der jeweilige Verstellweg der jeweils beförderten Strahlenquelle wird vom jeweiligen Impulsgeber erfasst, so dass die Strahlenquelle an die richtige Position im Tumor gebracht und auch exakt um gewünschte Schritte darin verschoben werden kann.

Die Antriebsmotoren A dienen dazu, bei Stromausfall die jeweilige Strahlenquelle aus dem Patienten herauszuziehen, da sie batteriegespeist sind. Sie erzeugen auch eine Vorspannung der Seile 5, 6, 7 und halten diese straff.

Der Aufbau gemäß Fig. 1 und 2 zeigt, dass die Rohre 1, 2, 3 rechts seitlich aus der Vorrichtung herausragen.

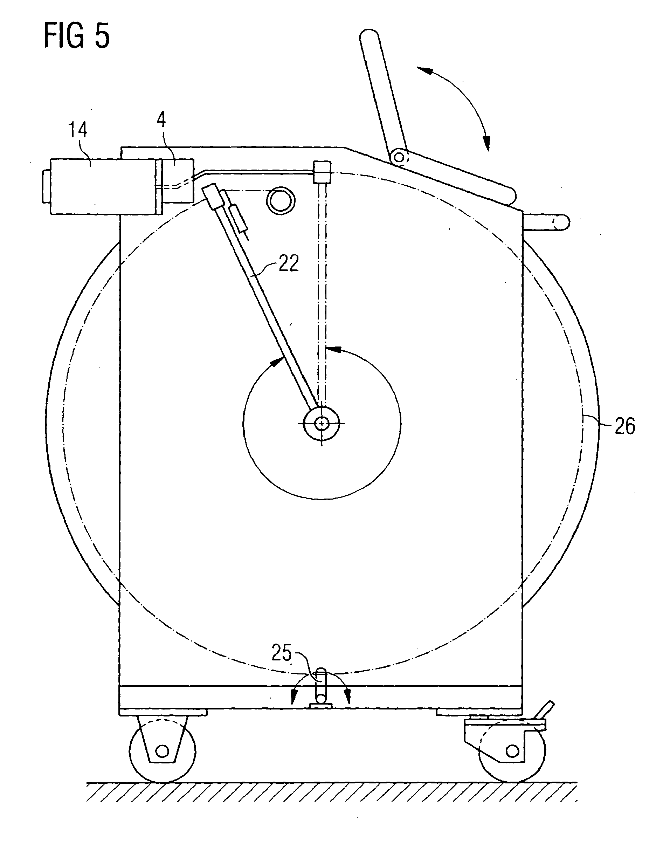

Einen symmetrischen Aufbau bezüglich des Fahrgestells zeigt die Fig. 5, bei der zwischen den beiden Enden jedes Rohres jeweils etwa ein Achtelkreis liegt. In Fig. 5 sind nicht alle Teile der Vorrichtung gezeigt und bezeichnet. Es ist hier ein fingerförmiger Rohrhalter 25 an der Unterseite der Rohre 1, 2, 3 (schematisch durch die gestrichelte Linie 26 verkörpert) angebracht, der die Rohre 1, 2, 3 umgreift. Er ist umklappbar, so dass der Halter 20 vorbei bewegt werden kann.

Ein besonders kompakter Aufbau ist in Fig.6 gezeigt. Hier ist schematisch ein spiralförmiges Rohr 27 dargestellt. An Stelle des starren Armes 22 ist ein Arm mit einem Schiebeglied 28 vorgesehen, der in seiner Länge dem jeweiligen Radius des spiralförmigen Rohres 27 anpassbar ist. An Stelle eines spiralförmigen Rohres kann auch ein wendelförmiges Rohr vorgesehen werden.

Die Fig.4 zeigt, dass auf dem Seil 7 mit dem Magnetelement M ein Stift 29 aus nicht magnetisierbarem Material verbunden ist, der eine Kerbe 30 aufweist, in die die Klemmvorrichtung 10 eingreift.

## Patentansprüche

1. Nachladevorrichtung zur Brachytherapie von Tumoren mit mindestens zwei Rohren (1, 2, 3) zur Aufnahme je eines längsverschiebbaren Seiles (5, 6, 7), an dessen einem Ende eine Strahlenquelle (12) anbringbar ist und mit einem abnehmbaren Verteiler (14), von dem Schläuche (15) ausgehen, die in den Tumor einführbar sind und der an einer Abschirmung (4) an einer Seite lösbar angebracht ist, an deren anderer Seite die Rohre (1, 2, 3) enden, wobei der Verteiler (14) derart verstellbar ist, dass in den Eingang des jeweils gewünschten Schlauches (15) die jeweils gewünschte Strahlenquelle (12) einführbar ist.

2. Nachladevorrichtung nach Anspruch 1, bei der an dem der Abschirmung (4) zugewandten Ende der Rohre (1, 2, 3) jeweils eine Quellen-Wechselvorrichtung (11) angebracht ist.

3. Nachladevorrichtung nach Anspruch 2, bei der die jeweilige Strahlenquelle (12) mit dem jeweiligen Seil (5, 6, 7) über eine Schraubverbindung (13) verbunden ist, deren einer Teil drehbar am Seilende angebracht ist.

4. Nachladevorrichtung nach einem der Ansprüche 1 bis 3, bei der jedem Seil (5, 6, 7) ein Impulsgeber (8) an dem der Abschirmung (4) abgewandten Ende zugeordnet ist, mit dem ein das Seil (5, 6, 7) tragender Spulenkörper (9) verbunden ist, der pro Umdrehung eine vorgestimmte Anzahl von Impulsen liefert.

5. Nachladevorrichtung nach Anspruch 4, bei der der Impulsgeber (8) mit einem Antriebsmotor (A) verbunden ist.

6. Nachladevorrichtung nach Anspruch 4 oder 5, bei der jedem Rohr (1, 2, 3) an dem dem Impulsgeber (8) zugeordneten Ende eine Klemmvorrichtung (10) zugeordnet ist, die das jeweils nicht zu verstellende Seil (5, 6, 7) festhält.

7. Nachladevorrichtung nach einem der Ansprüche 1 bis 6, bei der die Rohre (1, 2, 3) teilkreisförmig gekrümmt sind.

8. Vorrichtung nach Anspruch 7, wobei die Abschirmung (4) am oberen Ende der Nachladevorrichtung angebracht ist und die Seile (5, 6, 7) etwa horizontal zum Verteiler (14) führt.

9. Nachladevorrichtung nach Anspruch 7 oder 8, bei der jedes Rohr (1, 2, 3) von einem längs des Rohres (1, 2, 3) verschiebbaren Magneten (16, 17, 18) umschlossen ist, der auf ein magnetisch ankoppelbares Magnetelement (M) auf dem zugeordneten Seil (5, 6, 7) einwirkt und so das Seil (5, 6, 7) bei seiner Verschiebung auf dem Rohr (1, 2, 3) mitnimmt.

10. Nachladevorrichtung nach Anspruch 9, bei dem alle Magnete (16, 17, 18) in einem gemeinsamen Halter (20) gelagert sind, der am Ende eines um den Kreismittelpunkt (21) der Rohre (1, 2, 3) drehbaren Armes (22) befestigt ist.

11. Nachladevorrichtung nach Anspruch 10, bei der die Magnete (16, 17, 18) zum Ausgleich von Krümmungstoleranzen der Rohre (1, 2, 3) im Halter (20) quer zur Rohr-Längsrichtung verschiebbar gelagert sind.

12. Nachladevorrichtung nach Anspruch 10 oder 11, bei der an einer über den Drehpunkt des Armes (22) hinausragenden Verlängerung (23) des Armes (22) ein Ausgleichsgewicht (24) angebracht ist.

13. Nachladevorrichtung nach einem der Ansprüche 7 bis 12, bei der an der Unterseite ein fingerförmiger Rohrhalter (25) angebracht ist, der die Rohre (1, 2, 3) umgreift.

14. Nachladevorrichtung nach einem der Ansprüche 7 bis 13, bei der zwischen den beiden Ende jedes Rohres (1, 2, 3) jeweils etwa ein Viertelkreis liegt.

15. Nachladevorrichtung nach einem der Ansprüche 7 bis 13, bei der zwischen den beiden Enden jedes Rohres (1, 2, 3) jeweils etwa ein Achtelkreis liegt.

16. Nachladevorrichtung nach einem der Ansprüche 1 bis 6, bei der die Rohre (27) spiralförmig ausgeführt sind.

17. Nachladevorrichtung nach einem der Ansprüche 1 bis 15, bei der in einem der Rohre (1, 2, 3) ein Dummy untergebracht ist.

## Claims

1. Reloading device for the brachytherapy of tumours having at least two tubes (1, 2, 3), each for receiving a longitudinally displaceable cable (5, 6, 7), to whose one end a radiation source (12) may be applied and having a removable distributor (14), from which hoses (15) issue, which may be inserted into the tumour and which may be detachably mounted on a screen (4) on one side, on whose other side the tubes (1, 2, 3) end, the distributor (14) being so displaceable that the respectively desired radiation source (12) may be inserted into the input of the respectively desired hose (15).

2. Reloading device according to claim 1, wherein on the end of the tubes (1, 2, 3) associated with the screen (4) a respective source-changing device (11) is mounted.

3. Reloading device according to claim 2, wherein the respective radiation source (12) is connected to the respective cable (5, 6, 7) via a screw connection (13), whose one part is mounted rotatably on the cable end.

4. Reloading device according to one of claims 1 to 3, wherein a pulse generator (8) is allocated to each cable (5, 6, 7) at the end remote from the screen (4), to which pulse generator a coil body (9) bearing the cable (5, 6, 7) is connected and supplies a predetermined number of pulses per revolution.

5. Reloading device according to claim 4, wherein the pulse generator (8) is connected to a drive motor (A).

6. Reloading device according to claim 4 or 5, wherein a clamping device (10) is allocated to each tube (1, 2, 3) at the end associated with the pulse generator (8) and grips the cable (5, 6, 7) in each case not to be changed.

7. Reloading device according to one of claims 1 to 6, wherein the tubes (1, 2, 3) are curved into a partial circle.

8. Device according to claim 7, wherein the screen (4) is mounted at the upper end of the reloading device and guides the cables (5, 6, 7) roughly horizontally to the distributor (14).

9. Reloading device according to claim 7 or 8, wherein each tube (1, 2, 3) is surrounded by a magnet (16, 17, 18) which is displaceable along the tube (1, 2, 3) and which acts on a magnetic element (M) that may be coupled magnetically on the associated cable (5, 6, 7) and thus takes the cable (5, 6, 7) with it when displaced on the tube (1, 2, 3).

10. Reloading device according to claim 9, wherein all magnets (16, 17, 18) are mounted in a common holder (20) fixed to the end of an arm (22) rotatable about the centre of the circle (21) of the tubes (1, 2, 3).

11. Reloading device according to claim 10, wherein the magnets (16, 17, 18) are mounted displaceably transverse to the tube longitudinal direction in order to compensate tolerances in curvature of the tubes (1, 2, 3) in the holder (20).

12. Reloading device according to claim 10 or 11, wherein a balancing weight (24) is mounted on an extension (23) of the arm (22) projecting beyond the fulcrum of the arm (22).

13. Reloading device according to one of claims 7 to 12, wherein on the under-side a finger-like tube holder (25) is mounted, which encompasses the tubes (1, 2, 3).

14. Reloading device according to one of claims 7 to 13, wherein between the two ends of each tube (1, 2, 3) in each case there is roughly a quarter-circle.

15. Reloading device according to one of claims 7 to 13, wherein between the two ends of each tube (1, 2, 3) there is roughly an eighth of a circle.

16. Reloading device according to one of claims 1 to 6, wherein the tubes (27) are formed spirally.

17. Reloading device according to one of claims 1 to 15, wherein a dummy is housed in one of the tubes (1, 2, 3).

## Revendications

1. Dispositif de rechargement pour la brachythérapie de tumeurs comprenant au moins deux tubes (1, 2, 3) créés chacun pour le logement d'un câble (5, 6, 7) coulissant dans le sens longitudinal, sur une extrémité duquel une source de rayonnement (12) peut être placée, et un répartiteur (14) amovible, duquel partent des flexibles (15), pouvant être introduits dans la tumeur et qui est placé de façon amovible sur un blindage (4) sur un côté, sur l'autre côté duquel se terminent les tuyaux (1, 2, 3), le répartiteur (14) pouvant être déplacé de telle sorte que la source de rayonnement (12) respectivement souhaitée peut être introduite dans l'entrée du flexible (15) respectivement souhaité.

2. Dispositif de rechargement selon la revendication 1, sur lequel à chaque fois un dispositif de remplacement de source (11) est placé sur l'extrémité, associée au blindage (4), des tuyaux (1, 2, 3).

3. Dispositif de rechargement selon la revendication 2, sur lequel la source de rayonnement (12) respective est reliée au câble (5, 6, 7) respectif au moyen d'un assemblage vissé (13), dont une partie est placée de façon rotative sur l'extrémité du câble.

4. Dispositif de rechargement selon l'une quelconque des revendications 1 à 3, sur lequel à chaque câble (5, 6, 7) est attribué un générateur d'impulsion (8) sur l'extrémité opposée au blindage (4), auquel est relié un corps de bobine (9) portant le câble (5, 6, 7), lequel corps fournit pour chaque rotation un nombre prédéfini d'impulsions.

5. Dispositif de rechargement selon la revendication 4, sur lequel le générateur d'impulsion (8) est relié à un moteur d'entraînement (A).

6. Dispositif de rechargement selon la revendication 4 ou 5, sur lequel à chaque tuyau (1, 2, 3) est attribué sur l'extrémité associée au générateur d'impulsion (8) un dispositif de serrage (10) qui maintient le câble (5, 6, 7) concerné qui n'est pas à déplacer.

7. Dispositif de rechargement selon l'une quelconque des revendications 1 à 6, sur lequel les tuyaux (1, 2, 3) sont incurvés en forme de cercle partiel.

8. Dispositif selon la revendication 7, le blindage (4) étant placé sur l'extrémité supérieure du dispositif de rechargement et guidant les câbles (5, 6, 7) à peu près horizontalement vers le répartiteur (14).

9. Dispositif de rechargement selon la revendication 7 ou 8, sur lequel chaque tuyau (1, 2, 3) est entouré par un aimant (16, 17, 18) pouvant coulisser le long du tuyau (1, 2, 3), lequel aimant agit sur un élément magnétique (M) pouvant être couplé magnétiquement sur le câble (5, 6, 7) attribué et entraîne ainsi le câble (5, 6, 7) lors de son coulissement sur le tuyau (1, 2, 3).

10. Dispositif de rechargement selon la revendication 9, sur lequel tous les aimants (16, 17, 18) sont fixés dans un support (20) commun, qui est fixé sur l'extrémité d'un bras (22) pouvant tourner autour du centre du cercle (21) des tuyaux (1, 2, 3).

11. Dispositif de rechargement selon la revendication 10, sur lequel les aimants (16, 17, 18) destinés à la compensation de tolérances de courbure des tuyaux (1, 2, 3) sont fixés dans le support (20) de façon à pouvoir coulisser transversalement à la direction longitudinale du tuyau.

12. Dispositif de rechargement selon la revendication 10 ou 11, sur lequel un poids d'équilibrage (24) est placé sur un prolongement (23), dépassant du point de rotation du bras (22), du bras (22).

13. Dispositif de rechargement selon l'une quelconque des revendications 7 à 12, sur lequel est placé sur le côté inférieur un support de tuyau (25) en forme de doigt qui entoure les tuyaux (1, 2, 3).

14. Dispositif de rechargement selon l'une quelconque des revendications 7 à 13, sur lequel à chaque fois, à peu près un quart de cercle est disposé entre les deux extrémités de chaque tuyau (1, 2, 3).

15. Dispositif de rechargement selon l'une quelconque des revendications 7 à 13, sur lequel à chaque fois environ un huitième de cercle est disposé entre les deux extrémités de chaque tuyau (1, 2, 3).

16. Dispositif de rechargement selon l'une quelconque des revendications 1 à 6, sur lequel les tuyaux (27) sont réalisés en forme de spirale.

17. Dispositif de rechargement selon l'une quelconque des revendications 1 à 15, sur lequel un élément factice est logé dans l'un des tuyaux (1, 2, 3).
